# EUROPÄISCHE PATENTSCHRIFT

(11) **EP 2 750 788 B1**
(45) Veröffentlichungstag und Bekanntmachung des Hinweises auf die Patenterteilung: **12.06.2019**
(21) Anmeldenummer: 12766600.6
(22) Anmeldetag: 23.08.2012
(51) Int. Cl.: B01J 19/24, C07C 2/84

(54) **VERFAHREN ZUR OXIDATIVEN UMWANDLUNG VON ALKANEN UND REAKTORANORDNUNG ZUR DURCHFÜHRUNG DIESES VERFAHRENS**
METHOD FOR OXIDATIVELY CONVERTING ALKANES AND REACTOR ASSEMBLY FOR CARRYING OUT SAID METHOD
PROCÉDÉ DE TRANSFORMATION OXYDATIVE D'ALCANES ET ENSEMBLE RÉACTEUR POUR METTRE EN OEUVRE CE PROCÉDÉ

(30) Priorität: 02.09.2011 DE 102011082073
(43) Veröffentlichungstag der Anmeldung: 09.07.2014
(73) Patentinhaber: Technische Universität Berlin, 10623 Berlin (DE)
(72) Erfinder: ARELLANO-GARCIA, Harvey, 12157 Berlin (DE); GODINI, Hamid Reza, 10555 Berlin (DE); WOZNY, Günter, 16548 Glienicke (DE)
(74) Vertreter: Maikowski & Ninnemann Patentanwälte Partnerschaft mbB
(86) Internationale Anmeldenummer: PCT/EP2012/066416
(87) Internationale Veröffentlichungsnummer: WO 2013/030084

(56) Entgegenhaltungen:
- DE-T2- 69 824 620
- KIATKITTIPONG W ET AL: "Comparative study of oxidative coupling of methane modeling in various types of reactor", CHEMICAL ENGINEERING JOURNAL, ELSEVIER SEQUOIA, LAUSANNE, CH, Bd. 115, Nr. 1-2, 15. Dezember 2005 (2005-12-15), Seiten 63-71, XP027703019, ISSN: 1385-8947 [gefunden am 2005-12-15]
- TOTA ET AL: "Theoretical and Experimental Investigation of Concentration and Contact Time Effects in Membrane Reactors", CHEMICAL ENGINEERING RESEARCH AND DESIGN, PART A, INSTITUTION OF CHEMICAL ENGINEERS, XX, Bd. 82, Nr. 2, 1. Februar 2004 (2004-02-01), Seiten 236-244, XP022536966, ISSN: 0263-8762, DOI: 10.1205/026387604772992828

## Beschreibung

Die vorliegende Erfindung betrifft ein Verfahren zur oxidativen Umwandlung von Alkanen in einem Festbett-Membran-Reaktor nach Anspruch 1, eine Reaktoranordnung zur Durchführung dieses Verfahrens nach Anspruch 12 sowie eine Membran nach Anspruch 18.

Die oxidative Umwandlung von Alkanen umfasst die oxidative Kupplung von Methan als auch die oxidative Dehydrogenierung von höheren Alkanen.

Die oxidative Umwandlung von Alkanen, zum Beispiel die oxidative Kupplung von Methan (OCM), in welchem Alkane katalytisch oder ohne Katalysator in wertvolle Produkte, wie zum Beispiel Ethan und Ethen, umgesetzt werden, kann mittels einer Vielzahl von Verfahren durchgeführt werden. So wurde zum Beispiel die Anwendung von Membran-Reaktoren zur Durchführung des Verfahrens ausführlich untersucht.

Grundlage des oxidativen Kupplungsverfahrens ist die Oxidation von Methan gemäß der Gleichung

2 CH₄ + O₂ → C₂H₄ + 2 H₂O.

Diese Reaktion ist exothermisch und tritt üblicherweise bei hohen Temperaturen im Bereich zwischen 750 °C bis 950°C auf. Im Verlaufe der Reaktion wird das Alkan heterogen an einer Katalysatoroberfläche unter Ausbildung freier Alkyl-Radikale aktiviert, welche anschließend in der Gasphase miteinander unter Ausbildung höherer, längerkettiger Kohlenwasserstoffe z.B. Ethan oder nach Ethandehydrierung reagieren. Die Ausbeute dieser Reaktion ist im Allgemeinen durch nicht-selektive Reaktionen der Alkylradikale mit der Oberfläche und dem Sauerstoff in der Gasphase reduziert.

Aufgrund der großen Mengen an natürlichen Gasvorkommen und den immer steigenden Preisen von Öl und seinen Derivaten, wie Naphtalen und Ethylen, besteht seit längerem der Wunsch, ein direktes Verfahren zur Herstellung von höheren Olefinen aus natürlich vorkommenden Gasen zu entwickeln und zu implementieren. Mit dem OCM-Verfahren erfolgt die katalytische und homogene Umwandlung von Methan in wertvolle Produkte, meistens Olefine wie Ethen. Dieser Reaktionsprozess kann durch viele verschiedene Verfahren und Methoden umgesetzt werden. Es existieren ebenfalls Abwandlungen dieses Verfahrens, die den genannten OCM-Prozess als Teil eines allgemeinen Verfahrens zur Herstellung von Gasen beinhalten.

Die oxidative Umwandlung von Alkanen führt neben der Herstellung von den entsprechenden Umwandlungs- bzw. Kupplungsprodukten, wie höheren Alkanen und Alkenen, ebenfalls zu einer CO₂-Produktion, die die Verfahrenseffizienz drastisch reduziert. Somit ist die Bereitstellung von höheren Alkanprodukten mit hoher Ausbeute und Selektivität nach wie vor eine Herausforderung.

Um diese Herausforderung zu bewältigen, besteht eine Möglichkeit in der Kontrolle der Sauerstoffzufuhr. So wurde als eine Möglichkeit das Bewirken eines thermodynamischen Gleichgewichtes durch Austauschen von Edukten und Produkten während der Reaktion unter Verwendung von verschiedenen Reaktor-Operationsbedingungen untersucht.

Gemäß der Kinetik des oxidativen Umwandlungsverfahrens ist es möglich, die Konkurrenz zwischen der unerwünschten CO₂-Produktion und den gewünschten Reaktionen, wie zum Beispiel der Produktion von höherwertigen Alkanen, durch die Manipulierung der Zugänglichkeit der Ausgangsstoffe, wie zum Beispiel Sauerstoff zu beeinflussen.

Im Allgemeinen wird der größere Anteil des unerwünschten Reaktionsproduktes Kohlendioxid am Ende des Reaktors hergestellt, wo die höherwertigen Produkte, wie zum Beispiel Ethen, nach der oxidativen Umwandlung innerhalb des Reaktors zur Verfügung stehen. Der limitierende und definierende Parameter bei der Herstellung von Kohlendioxid ist die Zugänglichkeit des Sauerstoffs zum Reaktionsprodukt, wie Ethen. Darüber hinaus ist die Reaktionsordnung in Bezug auf den Ausgangsstoff, wie Methan, in den gewünschten Reaktionen jeweils höher, während die Reaktionsordnung in Bezug auf Sauerstoff in den gewünschten Reaktionen jeweils niedriger ist als die für die Kohlendioxidbildung. Zusätzlich wird die Ausbeute am Reaktoreingang, wo die Reaktionsraten hoch sind, stärker beeinflusst. Jeder Ansatz zur Kontrolle der Selektivität ist daher darauf gerichtet, die Konzentrationsprofile der Reaktanten und Produkte entlang des Reaktors bzw. der Reaktorlänge in geeignetem Maße zu beeinflussen.

Wie bereits erwähnt, ist die Verwendung von konventionellen Membran-Reaktoren in einem Verfahren der oxidativen Kupplung von Methan bekannt. In einem konventionellen Festbett-Membran-Reaktor wird Sauerstoff in die Mantelseite des Reaktors eingeführt und permeiert bzw. durchdringt die sich in dem Reaktor befindendliche Membran, zum Beispiel eine röhrenförmige Membran, die üblicherweise aus einem porösen Material besteht, um mit dem entsprechenden Alkan innerhalb der rohrförmigen Membran zu reagieren.

Somit werden die Fusion und Sauerstoffzugänglichkeit durch den partiellen Sauerstoffdruck in der Mantelseite kontrolliert. Auf diese Weise kann die Selektivität im Verhältnis zu den einfachen Festbettreaktoren erhöht werden.

In der DE 698 24 620 T2 wird z.B. ein katalytischer Membranreaktor zur Oxidation von Kohlenwasserstoffen eingesetzt. In dem beschriebenen Reaktor wird eine bevorzugt rohrenförmige Membran, insbesondere katalytische Membran, verwendet, die Sauerstoff oder ein sauerstoffhaltiges Gas von einem zu oxidierenden Reaktantengas trennt. Dabei werden Sauerstoff (O₂) oder andere sauerstoffhaltige Spezies an der äußeren Seite der Membran zu Sauerstoffanionen reduziert, die dann durch die Membran zu der inneren Seite derselbigen, die in Kontakt mit dem Reaktantengas steht, transportiert werden. Die rohrenförmige Membran ist mit einem Festbettkatalysator gefüllt, in welchem die Oxidation des jeweilig verwendeten Kohlenwasserstoffs erfolgt.

Jedoch weist diese Struktur bzw. Bedienung eines konventionellen Festbett-Membran-Reaktors noch weitere Möglichkeiten zur Verbesserung auf, da die Manipulierung des partiellen Sauerstoffdruckes auf der Mantelseite nur begrenzt möglich ist. Diese Begrenzung basiert im Wesentlichen auf dem Umstand, dass eine Erniedrigung des Sauerstoffpartialdruckes die Selektivität erhöht, sich jedoch gleichzeitig die Umsetzung von Methan oder anderen Kohlenwasserstoffen und damit die Ausbeute der Alkanherstellung drastisch erniedrigt.

Darüber hinaus ist eine gleichmäßige Sauerstoffzuführung entlang des Reaktors in der Mantelseite desselbigen nicht notwendigerweise kompatibel mit den Reaktionssequenzen des oxidativen Umwandlungsprozesses. In diesen Reaktionen wirkt sich eine gleichmäßige und hohe Sauerstoffzugänglichkeit insbesondere negativ auf die Selektivität der Umwandlungsreaktion am Reaktorende aus, wo einige Teile der höheren Kohlenwasserstoffe, wie zum Beispiel Ethan oder Ethen, zu Kohlendioxid umgesetzt werden. Zusätzlich weist der mantelseitige Gasstrom, auch als Strip-Phase bezeichnet, einen beträchtlichen Anteil an Sauerstoff und weiteren Produkten auf und kann daher nicht durch dieselbe Reaktorstruktur weiter verarbeitet werden.

Der vorliegenden Erfindung liegt somit die Aufgabe zugrunde, unter Berücksichtigung der obigen Ausführungen, ein alternatives Verfahren zur oxidativen Umwandlung von Alkanen, insbesondere zur oxidativen Kupplung von Methan, unter Verwendung eines Festbett-Membran-Reaktors, entweder als einzelner Reaktor oder in einem Reaktornetzwerk bereitzustellen, welches eine Erhöhung der Effizienz und Ausbeute des oxidativen Umwandlungsprozesses ermöglicht.

Diese Aufgabe wird erfindungsgemäß durch ein Verfahren mit den Merkmalen des Anspruchs 1 und eine Reaktoranordnung mit den Merkmalen des Anspruchs 12 gelöst.

Demnach wird ein Verfahren zur oxidativen Umwandlung von mindestens einem Alkan, bevorzugt einem C1-C4 Alkan, insbesondere bevorzugt von Methan, in mindestens einem ersten Festbett-Membran-Reaktor umfassend eine rohrförmige Membran mit einem als Reaktionsraum verwendeten Innenraum, wobei der Innenraum mit mindestens einem katalytischen Feststoff als Festbett gefüllt ist, einen die Membran umgebenden äußeren Reaktormantel und einem zwischen Reaktormantel und Membran ausgebildeten Mantelraum bereitgestellt. Der erfindungsgemäß verwendete erste Festbett-Membran-Reaktor wird vorliegend auch als PPBMR-Reaktor (Proposed Packed Bed Membrane Reactor) gekennzeichnet, d.h. dieser Reaktor wird entsprechend dem vorgeschlagenen erfindungsgemäßen Modus betrieben.

Das erfindungsgemäße Verfahren ist dadurch gekennzeichnet, dass mindestens ein Gasstrom enthaltend eine Mischung aus mindestens einem Alkan und Sauerstoff oder einem sauerstoffhaltigen Gas in den Innenraum der rohrförmigen Membran in den katalytischen Feststoff eingeleitet wird, z.B. umzusetzendes Alkan und Sauerstoff werden zusammen unmittelbar in den katalytischen Feststoff eingeleitet. Zusätzlich wird erfindungsgemäß mindestens ein Gasstrom enthaltend ein Alkan in den Mantelraum des Reaktors eingeleitet. Somit werden im Unterschied zu den bekannten Verfahren bzw. Fahrweisen von Festbett-Membran-Reaktoren die Reaktanten bestehend aus zu oxidierendem Alkan und Sauerstoff zusammen in einem Strom in das Katalysatorbett innerhalb der rohrförmigen Membran eingeleitet und nicht, wie herkömmlich, räumlich durch die Membran getrennt voneinander eingeleitet. Mit anderen Worten, das vorliegende Verfahren ist durch eine grundsätzlich andere Beschickungs- bzw. Zuführungsstrategie als in den herkömmlichen Verfahren angewandt gekennzeichnet.

Bevorzugt enthält das Festbett des Reaktors mindestens einen, für die oxidative Umwandlung geeigneten Katalysator. Das Festbett besteht also aus einem Katalysator als Oxidationszone innerhalb der röhrenförmigen Membran, der in Abhängigkeit vom jeweiligen Katalysatortyp in der Form der Katalysatorteilchen variieren kann und z.B. kugelförmig, unregelmäßig, zylinderförmig, geschichtet usw. vorliegen kann. Insbesondere besteht das Festbett aus einem dreidimensionalen Katalysator, der bevorzugt das Innere der Membranröhre ausfüllt.

In einer bevorzugten Ausführungsform des vorliegenden Verfahrens werden das mindestens eine Alkan und der Sauerstoff während des Durchleitens durch den katalytischen Feststoff zumindest teilweise zu einem ungesättigten Alkanprodukt umgesetzt. Das dabei gebildete Gasgemisch aus Alkan, Sauerstoff und ungesättigtem Alkanprodukt wird mit einem Druckgradienten zwischen 0,5 bar bis 2,0 bar, bevorzugt 0,7 bar bis 1,7 bar über die Membran aus dem katalytischen Feststoff und damit aus der Reaktionszone, insbesondere in den Mantelraum oder die Mantelzone in Form der sogenannten Strip-Phase abgeführt.

Es ist ebenfalls möglich, dass zumindest ein Teil der gebildeten oxidativen Umwandlungsprodukte, insbesondere höhere Alkane und Alkene, auch über die Membran in die Mantelseite des Reaktors diffundieren und von dort abgeleitet werden, d.h. es ist ebenfalls möglich, dass zumindest ein Teil der gewünschten Reaktionsprodukte über die Membran in den Mantelraum diffundieren.

Entlang des Reaktors werden die Reaktanten und Produkte von der Reaktionszone entsprechend ihres Konzentrationsgradienten entfernt. Damit werden diese Konzentrationen, insbesondere die Konzentration der Reaktanten, optimal in der Reaktionszone kontrolliert, um den Prozessverlauf in Bezug auf Ausbeute und Selektivität zu erhöhen.

Entsprechend werden am Reaktoranfang größtenteils Sauerstoff und die niederen Alkanausgangsstoffe, wie zum Beispiel Methan, entfernt, während die hergestellten höherwertigen Kohlenwasserstoffe, wie Alkane oder Alkene, bevorzugt am Reaktorende entfernt werden. Dies bietet die Möglichkeit einer höheren Sauerstoffkonzentration am Reaktoreingang, wodurch die absolute Ausbeute erhöht wird und auf der anderen Seite die Reaktionsprodukte und Sauerstoff aus den kritischen Reaktionszonen am Ende des Reaktors bzw. nahe zum Reaktorausgang entfernt werden, um somit die Selektivität zu erhöhen.

Die Entfernung der Reaktanten, wie Sauerstoff und höhere Kohlenwasserstoffe, wie Alkene, aus der Reaktionszone ist insbesondere am Reaktorende sehr effektiv, da das Reaktorende die kritische Zone in Bezug auf die konkurrierenden Reaktionen der CO₂- und Alken-Herstellung ist. Aus diesem Grund weist der vorliegende Verfahrensansatz eine höhere Kapazität in Bezug auf die Durchlassrate auf.

Der in das katalystische Festbett des ersten erfindungsgemäß betriebenen Festbett-Membran-Reaktors (PPBMR) einzuleitende Gasstrom kann ein Volumenverhältnis von Alkan, insbesondere Methan, zu Sauerstoff von 1 : 10, bevorzugt von 1 : 5 aufweisen. Des Weiteren können ein oder mehrere inerte Gase, wie z.B. Stickstoff oder Helium als Trägergase in einem Volumenanteil von 0 Vol% bis 80 Vol%, bevorzugt von 20 Vol% bis 60 Vol% in dem Gasstrom enthalten sein.

Wie oben ausgeführt, wird gemäß der vorliegenden Erfindung das mindestens eine Alkan zusätzlich in den Mantelraum des ersten Festbett-Membran-Reaktors (PPBMR), d.h. in den Raum zwischen der Außenseite der Membran und der Innenseite der Reaktorwand, eingeleitet. Dadurch wird die Zugänglichkeit des Alkans, insbesondere des Methans, als einer der Reaktanten in die Reaktionszone verbessert. Das zusätzlich auf der Mantelseite eingeführte Alkan kann so über die Membran verteilt in die Katalysatorzone dosiert werden, wodurch die Konzentration des Alkans in der Reaktionszone bei einer bestimmten, bevorzugt hohen Konzentration gehalten werden kann. Dadurch werden Ausbeute und Selektivität weiter erhöht.

Die im vorliegenden Verfahren verwendete Membran ist bevorzugterweise eine gasdurchlässige Membran, die z.B. mit einer Katalysatorschicht beschichtet sein kann. Der Gasstransport durch die Membran kann mittels Konvektion bzw. Diffusion oder elektrochemisch erfolgen. Das zur Beschichtung der Membran verwendete Material ist bevorzugt eine Siliziumhaltige Verbindung, insbesondere basierend auf kolloidalem Silizium bzw. Silizium-Sole und/oder einer Siliziumoxycarbid-Verbindung. Eine derartige Beschichtung von Membranen, wie z.B. den handelsüblichen Keramikmembranen erlaubt zum einen die Bereitstellung von Membranen mit der für den vorliegenden Prozess erforderlichen Porengrößenverteilung und andererseits wird die Thermostabilität der Keramikmembranen erhöht.

Die Porengröße des Membranmateriales liegt bei 2 nm bis 500 nm, bevorzugt 50 nm bis 200 nm für einen ersten erfindungsgemäß betriebenen Festbett-Membran-Reaktor (PPBMR) oder 2 nm bis 20 nm für einen zweiten konventionell betriebenen Festbett-Membran-Reaktor (Conventional Packed Bed Membrane Reactor, CPBMR), der weiter unten detaillierter beschrieben wird. Ebenfalls ist die Verwendung einer selektiven permeablen Membran vorstellbar.

Die Gasdurchlässigkeit der Membran wird durch mehrere Faktoren, wie z.B. Art des Katalysators, Einspeiserate, Dimension des Reaktors bestimmt. So kann der Permeatgasdurchfluss entsprechend der Gasdurchlässigkeit für den Sauerstofffluss im Falle des ersten erfindungsgemäß betriebenen Festbett-Membran-Reaktors (PPBMR) in einem Bereich zwischen 0.01 mol/m²s bis 1 mol/m²s für eine Membran mit einem Durchmesser von 10 mm liegen.

Der in dem vorliegenden Verfahren verwendete und erfindungsgemäß betriebene erste Festbett-Membran-Reaktor (PPBMR) kann in Form eines einzelligen Reaktors z.B. ein Rohreaktor mit einer röhrenförmigen Membran deren zwei Enden jeweils offen sind, betrieben werden. Es ist auch möglich, die Membran in Form einer sogenannten Dead-endStruktur anzuordnen, d.h. ein Ende der Membran ist geschlossen und weist keinen Auslass für die Gase auf.

Prinzipiell ist es auch denkbar, mehrere parallel zueinander verlaufende Membranröhren zu verwenden, die von einem Reaktormantel umgeben sind und die durch ein Verteilerrohr miteinander verbunden sind, in dem vorliegenden Verfahren einzusetzen.

Das vorliegende Verfahren kann mit einer Vielzahl von verschiedenen Verfahrensbedingungen betrieben werden. Die relevanten Verfahrensparameter sind dabei Temperatur, Druck, Sauerstoffkonzentration, Alkankonzentration, zum Beispiel Methankonzentration, Konzentration an inerten Gasen, das Verhältnis von Katalysatorgewicht und Flussrate, das Alkan / Sauerstoff-Verhältnis und Menge und Material der inerten Verpackung im Katalysatorbett. Die aufgeführten Verfahrensparameter werden insbesondere durch die umzusetzenden Alkane, der Art des Katalysators und der Dimension des Reaktors beeinflusst.

Bevorzugterweise wird das Verfahren bei Reaktionstemperaturen im Bereich zwischen 350°C und 1200°C, bevorzugt im Bereich zwischen 500°C und 1000°C, insbesondere bevorzugt zwischen 650°C und 900°C durchgeführt.

Der verfahrensgemäße Druck liegt in einem Bereich zwischen 0,2 bar und 100 bar, bevorzugt zwischen 1 bar und 20 bar, insbesondere bevorzugt zwischen 1 bar und 6 bar.

Als geeigneter Katalysator kann jeder für die oxidative Umwandlung von Kohlenwasserstoffen bekannte Katalysator aus der Gruppe der X_{P}Y_{R}Z_{S}Q_{T}/M_{A}O_{B}-Katalysatoren verwendet werden. Geeignete Katalysatoren sind z. B. in der US 4,754,091 aufgelistet. X, Y, Z und Q sind dabei bevorzugterweise ausgewählt aus den folgenden Elementen oder deren Oxide, Hydroxide und/oder Carbonate: Li, Na, K, Rb, Cs, Fr, Be, Mg, Ca, Sr, Ba, Y, Bi, Rh, Ru, La, Pb, W, Mn, AI, Ag, Au, Pt, Ir, Nb, Pd, Zr, Sb, Te, Ga, Ni, Cu, Sn, Fe, Co, Mo, Te, Cr, und/oder V. Die Indizes P, R, S und T reflektieren deren molares Verhältnis in dem Katalysator in dem Bereich von 0,001 bis 1. Die Summe der molaren Komponenten der unterschiedlichen Elemente in dem Katalysator muss jeweils 1 betragen. Als geeignete Katalysatoren sei beispielhafterweise auf die folgenden Katalysatoren hingewiesen: La₂O₃/CaO, Na-Mn-W/SiO₂, La-Sr/CaO, Li/MgO, Na/CaO, EaO/Ga₂O₃, La/MgO, La₂O₃-CeO₂, Sm₂O₃, Sm/La₂O₃.

Die Katalysatoren können ohne Träger oder mit einem Träger hergestellt aus einem Oxid des Typs M_{A}O_{B} verwendet werden, wobei M ausgewählt ist aus einer der folgenden Komponenten Mg, Ca, Sr, La, Zr, Si, AI, Y, Sm, Ti oder einem anderen keramischen Oxid-Trägermaterial. Des Weiteren können unterschiedliche inerte Packungen wie z. B. Quarzglas, Silizium, Aluminium, Magnesium, Titan und Gasverdünnungen durch z. B. Sauerstoff, Argon, Helium oder andere inerte Gase verwendet werden.

Die grundlegende Idee der vorliegenden Erfindung kann entweder in einem individuellen Reaktor oder in einer komplexen Reaktoranordnung bzw. einem Reaktornetzwerk enthaltend weitere Reaktortypen implementiert werden.

Entsprechend ist in einer Ausführungsform des vorliegenden Verfahrens dem oben beschriebenen erfindungsgemäß betriebenen ersten Festbett-Membran-Reaktor (PPBMR) mindestens ein Festbettreaktor (FBR) vorgeschaltet, so dass der Festbettreaktor und der erste erfindungsgemäß betriebene Festbett-Membran-Reaktor (PPBMR) in Reihe geschaltet sind. In diesen Festbettreaktor wird bevorzugt ein Gasstrom enthaltend eine Mischung aus mindestens einem Alkan und Sauerstoff oder einem sauerstoffhaltiges Gas zusammen eingeleitet.

In diesem, dem ersten Festbett-Membran-Reaktor (PPBMR) vorgeschalteten Festbettreaktor (FBR) wird bereits eine oxidative Umwandlung des Alkans eingeleitet. Auch hier wird die Umsatzrate durch die Dimension des verwendeten Reaktors, die Sauerstoffkonzentration, die Flussrate der eingespeisten Reaktanten und weiteren Faktoren beeinflusst. Festbettreaktoren (FBR) werden üblichwerweise zur Erhöhung der Alkan-, insbesondere Methanumsetzung bei kurzer Kontaktzeit verwendet, insbesondere wenn der eingespeiste Gasstrom frisch bzw. neu ist, und somit ausschließlich Alkane und keine Alkenprodukte z.B. durch Recycling enthält. Typischerweise trägt ein Festbett-Reaktor (FBR) zu 20 % bis 50 % der gesamten Alkanumsetzung bei.

Der den Festbettreaktor (FBR) verlassende Gasstrom enthaltend mindestens ein Alkan, Sauerstoff bzw. sauerstoffhaltiges Gas und mindestens ein Oxidationsprodukt des Alkans wird anschließend bevorzugt direkt bzw. unmittelbar in das Festbett aus dem katalytischen Feststoff des ersten Festbett-Membran-Reaktors (PPBMR) eingeleitet; der den FBR verlassende Gasstrom kann somit also auch als der oben definierte, in den Innenraum der rohrenförmigen Membran des ersten Festbett-Membran-Reaktors einzuleitende Gasstrom enthaltend ein Alkan und Sauerstoff bzw. sauerstoffhaltiges Gas angesehen werden.

In einer weiteren, bevorzugten Ausführungsform ist parallel zu dem in Reihe geschalteten Festbettreaktor und ersten, erfindungsgemäß betriebenen Festbett-Membran-Reaktor (PPBMR) mindestens ein zweiter, konventionell betriebener Festbett-Membran-Reaktor (CPBMR) geschaltet. In dieser Parallelschaltung wird bevorzugterweise mindestens ein Gasstrom, enthaltend mindestens ein Alkan, in das Festbett aus katalytischem Feststoff des zweiten Festbett-Membran-Reaktors (CPBMR) eingeleitet und ein Gasstrom enthaltend Sauerstoff oder ein sauerstoffhaltiges Gas in den Mantelraum des zweiten Festbett-Membran-Reaktors (CPBMR) eingeleitet.

Im Falle des zweiten, parallel geschalteten Festbett-Membran-Reaktors werden die Ausgangsstoffe bzw. Reaktanten somit getrennt in den Reaktor eingeführt, was bereits bekannt ist: der Gasstrom enthaltend den Kohlenwasserstoff bzw. Alkan wird direkt in das Katalysatorbett geleitet, während das Oxidationsmittel in Form von Sauerstoff oder sauerstoffhaltigem Gas in den Mantelraum und somit über die Membran verteilt in die Reaktionszone oder Katalysatorzone dosiert wird. Der zweite Festbett-Membran-Reaktor wird demnach konventionell nach dem bekannten Verfahren betrieben. Aus einer solchen Betriebsweise resultieren im Vergleich zur Betriebsweise des ersten Festbett-Membran-Reaktors geänderte Konzentrations- und Verweilzeitprofile der Reaktanten im Katalysatorbett.

Der in dem vorliegenden Verfahren verwendete Gasstrom aus gasförmigem Alkan kann vor Zufuhr in die Reaktoren zunächst in mindestens zwei Alkan-Teilsströme aufgeteilt werden. Dabei kann mindestens ein erster Alkan-Teilstrom mit Sauerstoff vermischt werden und anschließend in den mindestens einen Festbettreaktor (FBR) eingeleitet werden.

Der mindestens eine zweite Alkan-Teilstrom kann entweder ebenfalls mit frischem Sauerstoff, der z.B. über die Mantelseite der Membran des zweiten Festbett-Membran-Reaktors (CPBMR) eingeleitet wird, versetzt werden und/oder mit einem sauerstoffhaltigen Gasgemisch vermischt werden, das aus einem der anderen Reaktoren z.B. dem ersten Festbett-Membran-Reaktor (PPBMR) abgeleitet wird, und anschließend in das Katalysatorbett des zweiten Festbett-Membran-Reaktor (CPBMR) eingeleitet werden.

Es ist weiterhin besonders bevorzugt, wenn der mindestens eine zweite (Teil)gasstrom enthaltend das mindestens eine Alkan, der in das Katalysatorbett des zweiten Festbett-Membran-Reaktor (CPBMR) eingeleitet wird, mit dem aus dem ersten Festbett-Membran-Reaktor (PPBMR) abgeführten Gasgemisch (strip phase), enthaltend das mindestens eine nicht-umgesetzte Alkan, Sauerstoff und ungesättigte Alkanprodukt vor Einleiten in das Festbett des zweiten Festbett-Membran-Reaktors (CPBMR) gemischt wird.

Ebenfalls kann der in den Mantelraum des zweiten Festbett-Membran-Reaktors (CPBMR) eingeführte Gasstrom aus Sauerstoff oder sauerstoffhaltigem Gas nach Durchströmen bzw. Verlassen des Mantelraums des zweiten Festbett-Membran-Reaktors (CPBMR) als mantelseitiger Gasstrom mit dem den Festbettreaktor (FBR) verlassenden Gasgemisch enthaltend mindestens ein Alkan, Sauerstoff und mindestens ein Oxidationsprodukt des Alkans bzw. mindestens ein ungesättigtes Alkanprodukt gemischt werden und dieses Gasgemisch unmittelbar in das Festbett aus dem katalytischen Feststoff des ersten Festbett-Membran-Reaktors (PPBMR) eingeleitet werden.

Zu beachten ist, dass der in den Mantelraum des zweiten Festbett-Membran-Reaktors eingeführte Gasstrom enthaltend Sauerstoff oder sauerstoffhaltiges Gas während des Durchströmens des Mantelraumes zumindest teilweise aus dem Katalysatorbett des zweiten Festbett-Membran-Reaktors über die rohrförmige Membran in den Mantelraum diffundierendes Alkan und/oder ungesättigtes Alkanprodukt aufnimmt. Das den Mantelraum des zweiten Festbett-Membran-Reaktors verlassende Gasgemisch, d.h. der mantelseitige Gasstrom des zweiten Festbett-Membran-Reaktors, enthält somit bevorzugt Sauerstoff, und Mengen an Alkan und an ungesättigtem Alkanprodukt bzw. Alkanumwandlungsprodukt.

Es ist demnach ebenfalls möglich, dass der in den Mantelraum des zweiten Festbett-Membran-Reaktors (CPBMR) eingeführte Gasstrom aus Sauerstoff oder sauerstoffhaltigem Gas nach Durchströmen des Mantelraums und - wie oben beschrieben- nach Aufnahme von Alkan und ungesättigten Alkanprodukt und Verlassen des Mantelraums des zweiten Festbett-Membran-Reaktors (CPBMR) als mantelseitiger Gasstrom in die Rohrseite oder den Mantelraum des ersten Festbett-Membran-Reaktors (PPBMR) eingeleitet wird.

Somit erfolgt mit dem vorliegenden Verfahren ein mehrfaches Recycling bzw. eine mehrfache Wiederverwendung der eingesetzten Gase, wodurch eine Erhöhung der Gesamtausbeute erreicht wird.

Zum einen wird das aus dem Katalysatorbett des ersten Festbett-Membran-Reaktors (PPBMR) über die Membran in den Mantelraum des ersten Festbett-Membran-Reaktors (PPBMR) als Strip-Phase abgeführte Gasgemisch aus nicht-umgesetzten Alkan, Sauerstoff und ungesättigtem Alkanprodukt unmittelbar bzw. nach Vermischen mit weiterem, frischen Alkan in das Katalysatorbett des zweiten Festbett-Membran-Reaktors (CPBMR) eingeleitet und dort umgesetzt.

Zum anderen erfolgt eine mehrfache Verwendung des in den Mantelraum des zweiten Festbett-Membran-Reaktors (CPBMR) eingeführten Sauerstoffs. Dieser wird lediglich zu einem relativ kleinen Prozentsatz über die Membran des zweiten Festbett-Membran-Reaktors (CPBMR) in das Katalysatorbett verteilt. Der verbleibende überwiegende Teil des Sauerstoffs wird gemäß dem vorliegenden Verfahren nunmehr in den ersten Festbett-Membran-Reaktor (PPBMR) überführt. Da dieser Sauerstoffstrom nur zu einem geringen Anteil weitere Komponenten wie z.B. aus dem Katalysatorbett über die Membran abgeführtes Alkan, oxidiertes Alkan, Kohlenmonoxid oder Kohlendioxid enthält, ist eine weitere Verwendung durch Einleiten in den Mantelraum des ersten Festbett-Membran-Reaktors (PPBMR) und/oder ein Vermischen mit dem den Festbettreaktor (FBR) verlassenden Gasgemisch aus Alkan und Sauerstoff wünschenswert.

Das vorliegende Verfahren kann also auch in einer bevorzugten Ausführungsform in einer Reaktoranordnung durchgeführt werden, welche folgenden Komponenten bzw. Reaktoren umfasst: mindestens einen Festbettreaktor (FBR), mindestens einen ersten Festbett-Membran-Reaktor (PPBMR), der dem Festbettreaktor (PFR) nachgeschaltet ist, so dass Festbettreaktor (FBR) und erster Festbett-Membran-Reaktor (PPBMR) in Reihe geschaltet sind, und mindestens einen zweiten Festbett-Membran-Reaktor (CPBMR), der zu den in Reihe geschalteten Festbettreaktor (FBR) und ersten Festbett-Membran-Reaktor (PPBMR) parallel geschaltet ist.

Der Festbettreaktor (FBR) weist bevorzugt einen Einlass für einen Gasstrom enthaltend mindestens ein Alkan und Sauerstoff oder sauerstoffhaltiges Gas auf. Der zweite Festbett-Membran-Reaktor (CPBMR) weist ebenfalls einen Einlass für einen Gasstrom enthaltend mindestens ein Alkan auf, wobei das im Gasstrom für den Festbettreaktor (FBR) und im Gasstrom für den zweiten Festbett-Membran-Reaktor (CPBMR) enthaltende Alkan gleichen Ursprungs sind, d.h. das - wie oben beschrieben - der verwendete alkanhaltige Gasstrom in einen ersten Alkanteilstrom für den Festbettreaktor (FBR) und in einen zweiten Alkanteilstrom für den zweiten Festbett-Membran-Reaktor (CPBMR) aufgeteilt wird.

Die vorliegende Reaktoranordnung ermöglicht somit die Durchführung von verschiedenen Verfahren und unterschiedlichen Katalysatoren in der Mantel- und Rohrseite der Festbett-Membran-Reaktoren und daher eine Kombination von verschiedenen Prozessen.

Insbesondere umfasst die vorgeschlagene Reaktoranordnung bzw. das Reaktornetzwerk somit mindestens einen Reaktor des Typs Festbettreaktor (FBR) und mindestens zwei Festbett-Membran-Reaktoren (PPBMR, CPBMR). Dabei kann der Festbettreaktor (FBR) auch innerhalb des Membran-Reaktor-Modules angeordnet sein.

Die Reaktorsequenz basiert auf den folgenden Annahmen: zunächst muss der mantelseitige Strom von weiteren Reaktortypen verarbeitet werden. Die Produkte der Reaktionszone, das heißt, die Produkte, die aus dem Katalysatorbett innerhalb der röhrenförmigen Membran abgeführt werden, sollten eine ähnliche Zusammensetzung aufweisen, um ein einheitliches Produkt für das Netzwerk zur Verfügung zu stellen. Darüber hinaus ist es wünschenswert, das Potential eines Festbettreaktors (FBR) zur Erhöhung der Ausbeute auszunutzen und das Potential eines konventionell betriebenen Festbett-Membran-Reaktors (CPBMR) zur Erhöhung der Selektivität zu verwenden. Lediglich die vorgeschlagene Struktur des Verfahrens unter Verwendung des erfindungsgemäß betriebenen ersten Festbett-Membran-Reaktors (PPBMR) ermöglicht eine derartige Kombination.

Des Weiteren kann die Strip- Phase, die in dem vorliegenden Verfahren anfällt, durch einen weiteren Reaktortyp weiter prozessiert bzw. verarbeitet werden. Somit kann das vorliegende Verfahren zum Beispiel mit einem konventionellen Verfahren in einem Festbett-Membran-Reaktor verbunden werden. Auf diese Weise wird ein Netzwerk von Membranreaktoren zur Verfügung gestellt, das zu einer gleichzeitigen Ausbeuteerhöhung und Selektivitätssteigerung führt.

In der vorliegenden Reaktoranordnung ist der mindestens eine erste Festbett-Membran-Reaktor (PPBMR) mit dem mindestens einen zweiten Festbett-Membran-Reaktor (CPBMR) über mindestens eine Leitung gekoppelt bzw. verbunden.

In der vorliegenden Reaktoranordnung sind der mindestens einen erste Festbett-Membran-Reaktor (PPBMR) und der mindestens eine zweite Festbett-Membran-Reaktor (CPBMR) miteinander über zwei Leitungen gekoppelt bzw. verbunden.

Hierbei kann eine erste Leitung die Mantelseite bzw. den Mantelraum des ersten Festbett-Membran-Reaktors (PPBMR) unmittelbar mit einer Einströmleitung für das gasförmige Reaktionsgemisch in das Festbett des zweiten Festbett-Membran-Reaktor (CPBMR) koppeln bzw. verbinden. Hierdurch ist es möglich, die Strip-Phase aus dem Mantelraum des ersten Festbett-Membran-Reaktors (PPBMR) unmittelbar in das gasförmige Reaktionsgemisch für den zweiten Festbett-Membran-Reaktor (CPBMR) einzuleiten. Der erste und zweite Reaktor sind also miteinander durch mindestens eine Leitung bzw. Ableitung für die Strip-Phase aus dem ersten Festbett-Membran-Reaktor (PPBMR) gekoppelt.

Eine zweite Leitung zwischen beiden Festbett-Membran-Reaktoren ermöglicht die Ableitung des mantelseitigen Gasgemisches aus dem zweiten Festbett-Membran-Reaktor (CPBMR) entweder in die Mantelseite des ersten Festbett-Membran-Reaktors (PPBMR) oder bzw. zusätzlich eine Vermischung mit dem gasförmigen Reaktionsgemisch, das aus dem Festbettreaktor (FBR) unmittelbar in das katalytische Festbett des ersten Festbett-Membran-Reaktors (PPBMR) eingeleitet wird.

Diese Reaktoranordnung bzw. ein derartiges Reaktornetzwerk ermöglicht somit einen geschlossenen Gas- und Energiekreislauf und eine vorteilhafte Ausnutzung sowohl der Reaktionsstoffe als auch der durch die notwendige Erwärmung der Reaktionsstoffe aufgebrachten Energie.

Die vorliegende Erfindung wird detailliert unter Bezugnahme auf die Figuren und die folgenden Ausführungsbeispiele zum besseren Verständnis erklärt. Es zeigen:
- Figur 1: eine erste Ausführungsform des erfindungsgemäßen Verfahrens unter Verwendung eines ersten Festbett-Membran-Reaktors (PPBMR);
- Figur 2: eine zweite Ausführungsform des erfindungsgemäßen Verfahrens unter Verwendung eines Festbettreaktors (FBR) und eines ersten Festbett-Membran-Reaktors (PPBMR);
- Figur 3: eine dritte Ausführungsform des vorliegenden Verfahrens unter Verwendung eines Festbettreaktors (FBR), eines ersten Festbett-Membran-Reaktors (PPBMR) und eines zweiten Festbett-Membran-Reaktors (CPBMR);
- Figur 4: eine vierte Ausführungsform des vorliegenden Verfahrens unter Verwendung eines Festbettreaktors (FBR), eines ersten Festbett-Membran-Reaktors (PPBMR) und eines zweiten Festbett-Membran-Reaktors (CPBMR);
- Figuren 5A-D: Aufnahmen einer beschichteten Membran; und
- Figur 6: eine fünfte Ausführungsform des vorliegenden Verfahrens unter Verwendung eines Modules aus einem Festbettreaktors (FBR) und eines ersten Festbett-Membran-Reaktors (PPBMR).

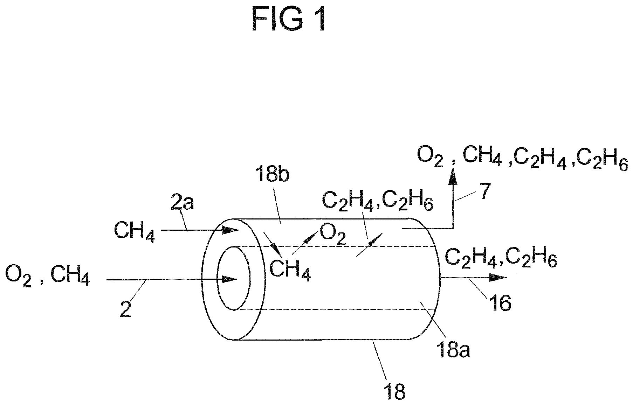

Figur 1 zeigt eine erste Ausführungsform des erfindungsgemäßen Verfahrens, bei welchem ein Gasstrom 2 enthaltend ein niederes Alkan wie z.B. Methan und Sauerstoff unmittelbar in den katalytischen Feststoff 18a als Festbett, welches aus dem in einer röhrenförmigen Membran (gestrichelt, siehe Figuren 1 bis 4) angeordneten Katalysators besteht, gemäß dem erfindungsgemäßen Verfahrens unter Verwendung eines ersten Festbett-Membran-Reaktors (PPBMR, 18) eingeleitet wird. Während des Durchflusses des Gasstroms durch das Reaktorbett bestehend aus dem katalytischen Feststoff 18a erfolgt die oxidative Kupplung des Methans unter Ausbildung eines nächst höheren Alkans wie z.B. Ethan C₂H₆ und/oder Alkens C₂H₄, die als Gasstrom 16 am Reaktorende abgeführt werden.

Zur Vermeidung von ungewünschten Seitenreaktionen der Alkan- und Alkenprodukte, insbesondere zu CO₂ wird ein bestimmter Prozentsatz der Ausgangsgemisches aus O₂ und Methan zusätzlich zu einem Anteil der Umwandlungsprodukte, aber insbesondere z.B. Ethen, Ethan und O₂ aus dem Reaktorbett in den Mantelraum 18b bzw. Mantelseite des Reaktors 18 abgeführt und verlässt den Mantelraum 18b als Teil des mantelseitigen Auslassstroms bzw. Gasgemisches 7. So wird eine unselektive Oxidation des gewünschten Reaktionsproduktes zu CO₂ am Reaktorende vermieden und damit die Ausbeute des Prozesses erhöht.

Zusätzlich wird vorliegend mantelseitig ein Alkanstrom 2a z.B. Methanstrom ggf. gemischt mit einem geeigneten inerten Gas in den Reaktor 18 eingeführt. Die mantelseitige Zufuhr von Alkan ermöglicht eine konstant hohe Konzentration des Alkans in der Reaktionszone des katalytischen Festbetts. Das mantelseitig eingeleitete Alkan, z.B. Methan diffundiert langsam entlang des Reaktors durch die Membran in das in der Membranröhre angeordnete Katalysatorbett, während Sauerstoff und ungesättigte Alkanprodukte wie z.B. Ethen oder gesättigte Alkanprodukte wie z.B: Ethan aus dem Katalysatorbett in die Mantelseite hinein diffundieren.

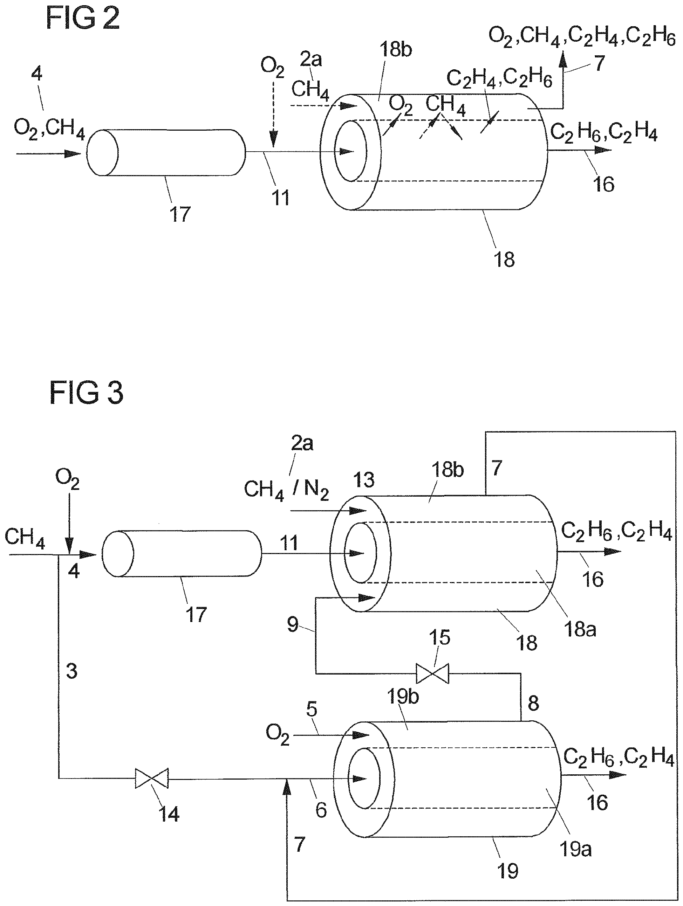

In der Ausführungsform gemäß Figur 2 ist dem ersten Festbett-Membran-Reaktors (PPBMR, 18) ein Festbettreaktor (FBR, 17) vorgeschaltet. Hierbei wird ein Gasstrom 4 enthaltend ein Alkan z.B. Methan und O₂ ggf. mit einem inerten Gasträger in den Festbettreaktor 17 eingeleitet. Das den Festbettreaktor 17 verlassende Gasgemisch 11 kann mit frischem Sauerstoff vermengt werden und wird anschließend in die Reaktorzone des Festbett-Membran-Reaktors (PPBMR, 18) zur weiteren Umsetzung zu höherwertigen, längerkettigen Kohlenwasserstoffen wie z.B. C₂H₄ eingeführt.

Das Vorschalten eines Festbettreaktors (FBR, 17) kann in Abhängigkeit von den angewendeten Verfahrensbedingungen die Reaktionsbelastung in dem ersten Membran-Festbettreaktor (PPBMR, 18) reduzieren. Da die für den Festbettreaktor notwendige Reaktorlänge für eine spezifische Methanumsetzung im Festbettreaktor geringer ist als die notwendige Reaktorlänge eines Festbett-Membran-Reaktors, kann bei Verwendung eines Festbettreaktors die benötigte Reaktorgesamtlänge reduziert werden. Dabei gibt es ein Optimum für die Länge und Leistung des Festbettreaktors.

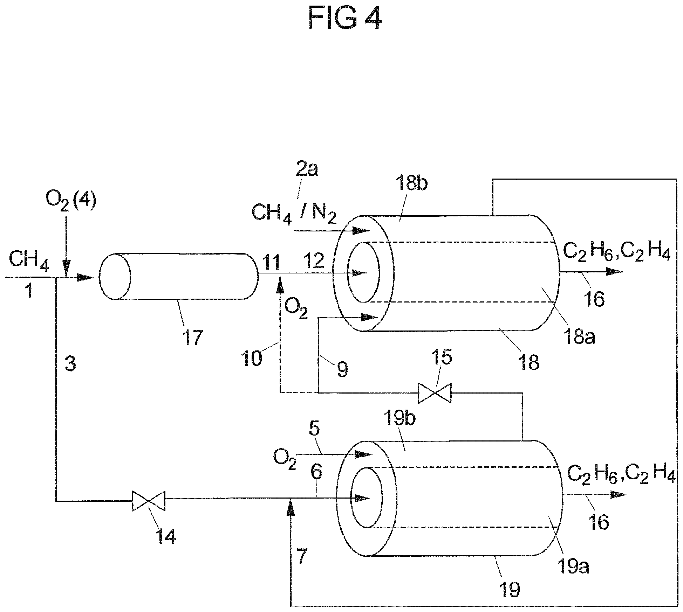

Figuren 3 und 4 zeigen schematisch weitere Ausführungsformen des vorliegenden Reaktornetzwerks. Hier wird der Ausgangsgasstrom enthaltend ein niederes Alkan, zum Beispiel C1-C4-Alkan, insbesondere Methan, zusammen mit 0 bis 80 % eines inerten Gases, wie Stickstoff, Helium oder weiteren geeigneten inerten Gasen, bevorzugt mit 20 bis 60 % des inerten Gases in das Netzwerk eingespeist. Der Einspeisestrom bzw. Ausgangsgasstrom wird in die zwei alkanhaltigen Teilströme 3 und 4 aufgeteilt, welche in dem vorgeschlagenen Festbettreaktor 17 (FBR) und anschließendem ersten Festbett-Membran-Reaktor 18 (PPBMR) und dem konventionellen Festbett-Membran-Reaktor 19 (CPBMR) verarbeitet werden. Der Sauerstoff wird in den ersten alkanhaltigen Teilgasstrom 4 für den Festbettreaktor 17 und ersten Festbett-Membran-Reaktor 18 eingeleitet und mit diesem vermischt. Der Festbettreaktor 17 ist dem ersten Festbett-Membran-Reaktor 18 vorgeschaltet.

In dem zweiten konventionell betriebenen Festbett-Membran-Reaktor 19 (CPBMR) wird der mantelseitige Sauerstoffstrom 5 mit dem Gasstrom aus der Reaktionszone 19a durch Membrandiffusion gemischt. Der Gasstrom 6, der in die Reaktionszone 19a des zweiten Reaktors 19 geleitet wird, setzt sich aus einer Kombination des mantelseitigen Gasstroms 7 des ersten Festbett-Membran-Reaktors 18 und des frischen zweiten alkanhaltigen Teilstroms 3 zusammen. Der mantelseitige Strom 8, der den zweiten Festbett-Membran-Reaktor 19 verlässt, kann als Reaktant 10 (siehe Figur 4) in der Reaktorzone des ersten Festbett-Membran-Reaktors 18 verwendet werden, oder kann als Gasstrom 9 in den Mantelraum 18b des ersten Festbett-Membran-Reaktors 18 eingeführt werden.

Der mantelseitige Strom 7, der den ersten Festbett-Membran-Reaktor 18 verlässt, weist einige Produkte, zum Beispiel Ethen auf, und wird daher, wie beschrieben, vor Einführung in die Reaktionszone des zweiten Festbett-Membran-Reaktors 19 mit dem frischen zweiten alkanhaltigen Teilgasstrom 3 gemischt. Das sich daraus ergebende Gasgemisch 6 kann dann weiter in der Reaktionszone 19a, das heißt in dem Katalysatorbett, angeordnet innerhalb der röhrenförmigen Membran des zweiten Festbett-Membran-Reaktors 19, verarbeitet werden.

In Abhängigkeit von der Zusammensetzung des Mantelstromes 10 des zweiten Festbett-Membran-Reaktors 19 und des Ausgangsstromes 11 des Festbettreaktors 17 wird die Zusammensetzung des Gasstromes 12, der in die Reaktionszone 18a, das heißt in das Katalysatorbett 18a innerhalb der röhrenförmigen Membran des ersten Festbett-Membran-Reaktors 18 eingeleitet wird, bestimmt. Dieser in die Reaktionszone 18a einzuführende Gasstrom steht einem mantelseitigen Strom 13, enthaltend Stickstoff und Methan, gegenüber.

Die Zusammensetzung des Mantelstroms 8, der den zweiten Festbett-Membran-Reaktor 19 verlässt, sowie die in dem zweiten Festbett-Membran-Reaktor 19 eintretende Gasmischung 6 wird über zwei Drosselventile 14, 15 eingestellt, um so die entsprechende Diffusion über die Membran des zweiten Festbett-Membran-Reaktors 19 zu kontrollieren und somit auch die Menge des frisch in den zweiten Reaktor 19 einzuführenden Gasstromes 6 einzustellen.

Die Produktströme 16, die die beiden Membranreaktoren 18, 19 verlassen, weisen bevorzugterweise die gleiche Zusammensetzung auf.

Die grundlegenden Blöcke in diesem Netzwerk sind die Reaktoren 17, 18, 19, in welchen die mantelseitigen Gasströme und die Gasströme aus den Reaktionszonen sich in einem Gegenstromverfahren und bevorzugterweise in einem Gleichflussverfahren treffen.

Die vorliegende Erfindung kann für parallele Reaktionen in der Gas- und/oder Flüssigphase mit unterschiedlichen Membranmaterialien und Membrandimensionen verwendet werden. Unterschiedliche Reaktionen können in den unterschiedlichen Reaktorblöcken durchgeführt werden.

### Ausführungsbeispiel 1: Modifizierte Membran

Kommerziell erhältliche Mikrofiltrationsmembranen, die üblicherweise zum Einsatz kommen, weisen nicht die notwendige Durchlässigkeit auf und können somit nicht in den beschriebenen Membranreaktoren verwendet werden. So ist z. B. eine Membran mit einer dünnen stabilen Membranschicht mit sehr kleinen Porengrößen notwendig, um eine feine Dosierung der Reaktanten zu ermöglichen und so den starken radialen Konzentrationsgradienten der Reaktanten bereitzustellen, um die Reaktanten außerhalb und innerhalb der Membranröhre zu trennen.

Derzeit erhältliche Keramikmembranen sind entweder nicht stabil bei den hohen Temperaturen zwischen 750°C bis 900 °C oder weisen nicht die erforderliche Nanoporengrößeverteilung auf.

Aus diesem Grund ist es notwendig, die derzeitigen Keramikmembranen zu modifizieren. So wurde eine stabile alpha-Aluminiummembranschicht auf einer Keramikmembran, die eine Porengröße von ca. 200 nm aufweist, modifiziert, um die erforderliche stabile Porengröße von weniger als 10 nm zu erhalten und somit die notwendige Permeabilität zu erhalten.

Dazu wurde eine kommerziell erhältliche Mikrofiltration alpha-Aluminiummembran mit kolloidalem Silizium imprägniert und anschließend kalziniert. Darüber hinaus ist es möglich, die Membran mit einer Siliziumoxicarbid (SiOC) Zusammensetzungzu beschichten.

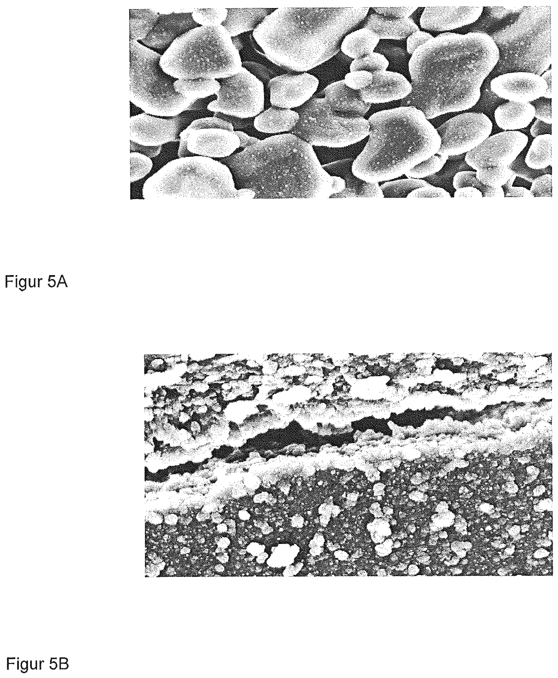

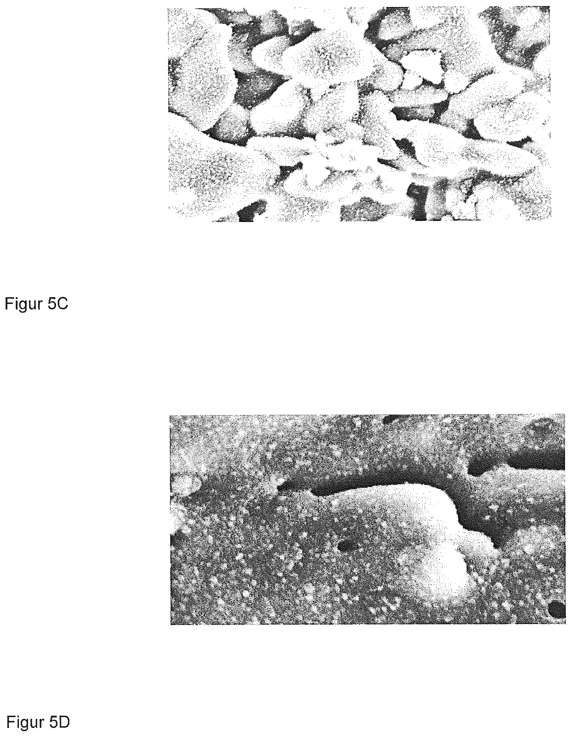

In den Figuren 5A bis 5C sind Aufnahmen der beschichteten Membran mittels Oberflächenelektronenmikroskopie (SEM) gezeigt. So zeigt Figur 5A die Aufnahme einer unbeschichteten bzw. unbehandelten Keramikmembran, die üblicherweise mit alpha-Aluminium beschichtet ist. In den Figuren 5B und 5C sind jeweils Membranoberflächen beschichtet mit kolloidalem Silizium (Figur 5B) und Siliziumoxycarbid (Figur 5C) gezeigt. Figur 5D wiederum zeigt eine Kombination der beschriebenen Beschichtungsverfahren, d. h., eine Kombination aus kolloidalem Silizium und Siliziumoxycarbid.

Wie der Aufnahme der Figur 5B zu entnehmen ist, führt die Beschichtung mit kolloidalem Silizium typischerweise zur Ausbildung von Spalten bzw. Rissen über der Oberfläche nach Kalzinierung. Hingegen zeigt eine mit Siliziumoxycarbid beschichtete Membran eine homogene Oberflächenbedeckung bzw. Oberflächenbeschichtung (siehe Fig. 5C). Durch die Kombination von Imprägnierung-Kalzinierung mit einem sequenziellen Dip-Coating der Membran in einer Zusammensetzung aus Siliziumoxicarbid (SiOC) werden bessere Eigenschaften der Oberflächenbeschichtung erreicht (siehe Fig. 5D).

Tabelle 1 vergleicht die Membraneigenschaften von unmodifizierten Membranen mit denen von mit unterschiedlichen Methoden beschichteten Membranen. Wie ersichtlich ist es möglich, durch eine Beschichtung mit kolloidalem Silizium bzw. Siliziumoxycarbid eine Reduzierung des Porenvolumens, des Porendurchmessers und des Oberflächenbereiches bzw. der spezifischen Oberfläche zu erreichen. Solche Modifizierungmethoden sind sehr entscheidend, um verschiedene Membranreaktoren mit der Durchlässigkeit entsprechend den hier verwendeten CPBMR und PPBMR's zu erhalten.

**Tabelle 1: Vergleich der Membraneigenschaften von Membranen mit unterschiedlichen Beschichtungen**

| **Art der Membran** | **Porenvolumen [cc/g]** | **Spez. Oberfläche [m2/g]** | **Porendurchmesser [nm]** |
|---|---|---|---|
| Unbehandelt | 0,44 | 99,66 | 200 (nominal) |
| Zweifach mit kolloidalen Silizizm beschichtet | 0,012 | 3,9 | 6,3 |
| Zweifach mit SiOC beschichtet | 0,003 | 1,5 | 2,2 |
| Vierfach mit kolloidalen Silizium und zweifach mit SiOC beschichtet | 0,003 | 1,3 | 2 |

Auch durch eine komplette Blockierung der Membranporen, kann ein Rohr mit einer Membran mit geringer oder keiner Durchlässigkeit hergestellt werden.

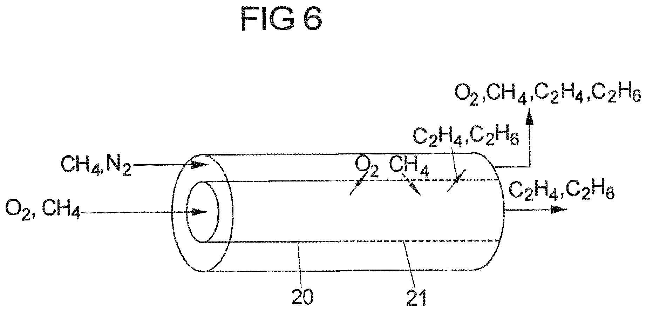

Es ist z.B. vorstellbar (Figur 6), dass die Membranporen in einem ersten Teil bzw. Abschnitt der Membran 20 mit der beschriebenen Modifizierungsmethode komplett blockiert werden und in einem zweiten Teil bzw. Abschnitt der Membran 21 die Porengröße entsprechend den Erfordernissen wie oben beschrieben modifiziert werden.

Solch eine behandelte Membranröhre fungiert dann in dem ersten, komplett blockierten Abschnitt wie ein Festbettreaktor (FBR) und in dem zweiten Abschnitt wie ein erfindungsgemäß betriebener Festbett-Membran-Reaktor (PPBMR, siehe Figur 6). Ein derartiger Aufbau vereint somit in einem einzigen Reaktor einen Festbettreaktor (FBR) im Bereich der blockierten Membran 20 und einen Festbett-Membran-Reaktor (PPBMR) im Bereich der durchlässigen modifizierten Membran 21.

In einer derartigen Anordnung kühlt der mantelseitige Gasstrom aus CH₄ und N₂ den FBR und erniedrigt so eine "Hotspotformation" im Katalysatorbett.

### Ausführungsbeispiel 2: Reaktoranordnung

Die gemäß Ausführungsbeispiel 1 modifizierte Keramikmembran wurde anschließend in einem Festbett-Membran-Reaktor getestet.

Zum Nachweis der Synthesekapazität der oben beschriebenen Reaktoren und Netzwerkstrukturen wurden verschiedene objektive Funktionsparameter untersucht und bestimmt. Als geeignete Funktionsparameter wurden die Selektivität, Methan-Konversion, C2 Ausbeute und der kombinierte Wert von Selektivität und Methan-Konversion für die einzelnen Reaktoren und die Netzwerkstruktur bestimmt.

Die Ergebnisse der mehrfachen Optimierung, die auf Grundlage von Modell basierten Untersuchungen durchgeführt und experimentell bestätig wurde, sind in den Tabellen 1 und 2 angeführt.

**Tabelle 1: oxidative Methankopplung durchgeführt in einem konventionell betriebenen Festbett-Membran-Reaktor (CPBMR)**

| **Objektiver Funktionsparameter** | **5** | **4** | **3** | **2** | **1** |
|---|---|---|---|---|---|
| Selektivität (%) | 85.2 | 99.97 | 70.15 | 82.09 | 82.09 |
| Methan Umwandlung (%) | 32.15 | 5.01 | 43.08 | 40.42 | 40.42 |
| C2 Ausbeute (%) | 27.4 | 5.01 | 30.22 | 33.18 | 33.18 |
| Selektivität + Methan Umwandlung (%) | 117.35 | 104.98 | 113.23 | 122.51 | 122.51 |

**Tabelle 2: oxidative Methankopplung durchgeführt in einem Netzwerk bestehend aus PPBMR, CPBMR und FBR**

| **Objektiver Funktionsparameter** | **5** | **4** | **3** | **2** | **1** |
|---|---|---|---|---|---|
| CPBMR Länge (mm) | 20 | 2 | 70 | 70 | 70 |
| FBR Länge (mm) | 2 | 10 | 10 | 10 | 10 |
| PPBMR Länge (mm) | 40 | 100 | 100 | 100 | 100 |
| Selektivität (%) | 93.35 | 96.86 | 88.51 | 88.51 | 88.51 |
| Methan Umwandlung (%) | 30.19 | 3.9 | 35.21 | 35.21 | 35.21 |
| C2 Ausbeute (%) | 28.18 | 3.77 | 31.17 | 31.17 | 31.17 |
| Selektivität + Methan Umwandlung (%) | 123.54 | 100.7 | 123.72 | 123.72 | 123.72 |

Wie aus den in Tabelle 1 und 2 angeführten Daten ablesbar, ist die Leistung der Netzwerkstruktur gegenüber den einzelnen Membran-Reaktoren, insbesondere CPBMR, verbessert. Bei Betrachtung der fünf, vorliegend angewandten Vergleichskriterien kann die Netzwerkstruktur drei dieser Kriterien gleichzeitig erfüllen.

Die vorliegende Netzwerkstruktur kann nur unter Verwendung des erfindungsgemäß betriebenen ersten Festbett-Membran-Reaktors (PPBMR) entwickelt werden.

## Patentansprüche

1. Verfahren zur oxidativen Umwandlung von mindestens einem Alkan, bevorzugt einem C1-C4 Alkan, insbesondere bevorzugt von Methan, in mindestens einem ersten Festbett-Membran-Reaktor (PPBMR, 18) umfassend eine rohrförmige Membran mit einem Innenraum als Reaktionsraum, wobei der Innenraum mit mindestens einem katalytischen Feststoff (18a) als Festbett gefüllt ist, einen die Membran umgebenden äußeren Reaktormantel und einem zwischen Reaktormantel und Membran ausgebildeten Mantelraum (18b),
**dadurch gekennzeichnet, dass**
mindestens ein Gasstrom (2, 11, 12) enthaltend eine Mischung aus mindestens einem Alkan und Sauerstoff oder einem sauerstoffhaltigen Gas in den Innenraum der rohrförmigen Membran in den katalytischen Feststoff (18a) eingeleitet wird und zusätzlich mindestens ein Gasstrom (2a) enthaltend mindestens ein Alkan in den Mantelraum (18b) eingeleitet wird.

2. Verfahren nach Anspruch 1, **dadurch gekennzeichnet, dass** das mindestens eine Alkan in der Gegenwart des Sauerstoffs während des Durchleitens durch den katalytischen Feststoff (18a) zumindest teilweise zu einem ungesättigten Alkanprodukt umgesetzt wird und das dabei gebildete Gasgemisch (7) aus Alkan, Sauerstoff und ungesättigtem Alkanprodukt über die Membran aus dem katalytischen Festbett (18a) und damit aus dem Reaktionsraum in den Mantelraum (18b) abgeführt wird.

3. Verfahren nach Anspruch 1 oder 2, **dadurch gekennzeichnet, dass** das Volumenverhältnis von Alkan, insbesondere Methan und Sauerstoff im Gasstrom 1 : 10, bevorzugt 1 : 5, insbesondere bevorzugt 1: 4 beträgt.

4. Verfahren nach einem der vorhergehenden Ansprüche, **dadurch gekennzeichnet, dass** dem ersten Festbett-Membran-Reaktor (PPBMR, 18) mindestens ein Festbettreaktor (FBR, 17) vorgeschaltet ist, so dass der mindestens eine Festbettreaktor (FBR, 17) und der erste Festbett-Membran-Reaktor (PPBMR, 18) in Reihe geschaltet sind.

5. Verfahren nach Anspruch 4, **dadurch gekennzeichnet, dass** ein Gasstrom (4) enthaltend eine Mischung aus mindestens einem Alkan und Sauerstoff oder einem sauerstoffhaltigen Gas in den mindestens einen Festbettreaktor (FBR, 17) eingeleitet wird.

6. Verfahren nach Anspruch 4 oder 5, **dadurch gekennzeichnet, dass** der den Festbettreaktor (FBR, 17) verlassende Gasstrom (11) enthaltend mindestens ein Alkan, Sauerstoff und mindestens ein Umwandlungsprodukt des Alkans in den Innenraum der rohrförmigen Membran in das Festbett (18a) aus dem katalytischen Feststoff des ersten Festbett-Membran-Reaktors (PPBMR, 18) eingeleitet wird.

7. Verfahren nach einem der vorhergehenden Ansprüche, **dadurch gekennzeichnet, dass** parallel zu den in Reihe geschalteten Festbettreaktor (FBR, 17) und dem ersten Festbett-Membran-Reaktor (PPBMR, 18) mindestens ein zweiter Festbett-Membran-Reaktor (CPBMR, 19) geschaltet ist.

8. Verfahren nach Anspruch 7, **dadurch gekennzeichnet, dass** mindestens ein Gasstrom (3) enthaltend mindestens ein Alkan in das Festbett (19a) aus katalytischen Feststoffs des zweiten Festbett-Membran-Reaktors (CPBMR, 19) eingeleitet wird und ein Gasstrom (5) aus Sauerstoff oder ein sauerstoffhaltigen Gas in den Mantelraum (19b) des zweiten Festbett-Membran-Reaktors (CPBMR, 19) eingeleitet wird.

9. Verfahren nach Anspruch 7 oder 8, **dadurch gekennzeichnet, dass** der mindestens eine Gasstrom (3) enthaltend das mindestens eine Alkan , der in das Festbett (19a) des zweiten Festbett-Membran-Reaktors (CPBMR, 19) eingeleitet wird, mit dem aus dem ersten Festbett-Membran-Reaktor (PPBMR, 18) abgeführten Gasgemisch (strip phase, 7) enthaltend das mindestens eine nicht- umgesetzte Alkan, ein ungesättigtes Alkanprodukt und Sauerstoff vor Einleiten in das Festbett (19a) des zweiten Festbett-Membran-Reaktor (CPBMR, 19) gemischt wird.

10. Verfahren nach einem der Ansprüche 7 bis 9, **dadurch gekennzeichnet, dass** der in den Mantelraum (19b) des zweiten Festbett-Membran-Reaktors (CPBMR, 19) eingeführte Gasstrom (5) aus Sauerstoff oder sauerstoffhaltigen Gas nach Durchströmen und Verlassen des Mantelraums (19b) des zweiten Festbett-Membran-Reaktors (CPBMR, 19) als mantelseitiger Gasstrom (8) mit dem den Festbettreaktor (FBR, 17) verlassenden Gasstrom (11) enthaltend mindestens ein Alkan, Sauerstoff und mindestens ein Umwandlungsprodukt des Alkans gemischt wird und dieser Gasstrom (12) in den Innenraum der rohrförmigen Membran in das Festbett (18a) aus dem katalytischen Feststoff des ersten Festbett-Membran-Reaktors (PPBMR, 18) eingeleitet wird.

11. Verfahren nach einem der Ansprüche 7 bis 10, **dadurch gekennzeichnet, dass** der in den Mantelraum (19b) des zweiten Festbett-Membran-Reaktors (CPBMR, 19) eingeführte Gasstrom (5) aus Sauerstoff oder sauerstoffhaltigen Gas nach Durchströmen und Verlassen des Mantelraums (19b) als mantelseitiger Gasstrom (8) des zweiten Festbett-Membran-Reaktors (CPBMR, 19) in den Mantelraum (18b) des ersten Festbett-Membran-Reaktors (PPBMR, 18) eingeleitet wird.

12. Reaktoranordnung zur Durchführung eines Verfahrens nach einem der vorhergehenden Ansprüche
**gekennzeichnet durch**
- mindestens einen Festbettreaktor (FBR, 17),
- mindestens einen ersten Festbett-Membran-Reaktor (PPBMR, 18) umfassend eine rohrförmige Membran mit einem Innenraum als Reaktionsraum, wobei der Innenraum mit mindestens einem katalytischen Feststoff (18a) als Festbett gefüllt ist, einen die Membran umgebenden äußeren Reaktormantel und einem zwischen Reaktormantel und Membran ausgebildeten Mantelraum (18b), wobei dieser erste Festbett-Membran-Reaktor (PPBMR, 18) dem Festbettreaktor (FBR, 17) nachgeschaltet ist, so dass Festbettreaktor (FBR, 17) und erster Festbett-Membran-Reaktor (PPBMR, 18) in Reihe geschaltet sind , und
- mindestens einen zweiten Festbett-Membran-Reaktor (CPBMR, 19), der zu dem in Reihe geschalteten Festbettreaktor (FBR, 17) und ersten Festbett-Membran-Reaktor (PPBMR, 18) parallel geschaltet ist,
- wobei der mindestens eine erste Festbett-Membran-Reaktor (PPBMR, 18) mit dem mindestens einen zweiten Festbett-Membran-Reaktor (CPBMR, 19) über mindestens eine erste Leitung gekoppelt ist, wobei durch diese erste Leistung der mantelseitige Strom (7) aus dem ersten Festbett-Membran-Reaktors (PPBMR, 18) in die Leitung für den Gasstrom (3) enthaltend mindestens ein Alkan für den zweiten Festbett-Membran-Reaktor (CPBMR, 19) eingeleitet wird, und
- wobei eine zweite Leitung zwischen beiden Festbett-Membran-Reaktoren (18, 19) vorgesehen ist, wobei durch die zweite Leitung der mantelseitige Gasstrom (8) aus dem zweiten Festbett-Membran-Reaktor (CPBMR, 19) in die Mantelseite (18b) des ersten Festbett-Membran-Reaktors (PPBMR, 18) und/oder in die Leitung für den den Festbettreaktor (FBR, 17) verlassenden Gasstrom (11) in das katalytische Festbett (18a) des ersten Festbett-Membran-Reaktors (PPBMR, 18) eingeleitet wird.

13. Reaktoranordnung nach Anspruch 12, **dadurch gekennzeichnet, dass** der erste Festbett-Membran-Reaktor (PPBMR, 18) und/oder der zweite Festbett-Membran-Reaktor (CPBMR, 19) mindestens eine rohrförmige, gasdurchlässige Membran mit einer Porengröße von 2 nm bis 500 nm, bevorzugt 50 nm bis 200 nm für den ersten Festbett-Membran-Reaktor (PPBMR, 18) oder 2 nm bis 20 nm für den zweiten Festbett-Membran-Reaktor (CPBMR, 19) aufweisen.

14. Reaktoranordnung nach einem der Ansprüche 12 bis 13, **dadurch gekennzeichnet, dass** der erste Festbett-Membran-Reaktor (PPBMR, 18) und/oder der zweite Festbett-Membran-Reaktor (CPBMR, 19) mindestens eine rohrförmige, gasdurchlässige Membran aufweisen, die mit einer siliziumhaltigen Verbindung beschichtet ist.

15. Reaktoranordnung nach Anspruch 14, **dadurch gekennzeichnet, dass** das zur Beschichtung der Membran verwendete Material eine Siliziumhaltige Verbindung, insbesondere basierend auf kolloidalem Silizium bzw. Silizium-Sole und/oder einer Siliziumoxycarbid-Verbindung ist.

## Claims

1. Process for oxidative conversion of at least one alkane, preferably of a C1-C4 alkane, especially preferably of methane, in at least one first fixed bed membrane reactor (PPBMR, 18) comprising a tubular membrane having an interior as the reaction space, wherein the interior is filled with at least one catalytic solid (18a) as a fixed bed, an outer reactor shell surrounding the membrane and a shell space (18b) formed between the reactor shell and the membrane,
**characterized in that**
at least one gas stream (2, 11, 12) containing a mixture of at least one alkane and oxygen or an oxygen-containing gas is introduced into the interior of the tubular membrane into the catalytic solid (18a) and additionally at least one gas stream (2a) containing at least one alkane is introduced into the shell space (18b).

2. Process according to Claim 1, **characterized in that** the at least one alkane is at least partially converted into an unsaturated alkane product in the presence of the oxygen during passage through the catalytic solid (18a) and the thus-formed gas mixture (7) of alkane, oxygen and unsaturated alkane product is discharged through the membrane out of the catalytic fixed bed (18a) and thus out of the reaction space into the shell space (18b).

3. Process according to Claim 1 or 2, **characterized in that** the volume ratio of alkane, in particular methane, and oxygen in the gas stream is 1:10, preferably 1:5, especially preferably 1:4.

4. Process according to any of the preceding claims, **characterized in that** the first fixed bed membrane reactor (PPBMR, 18) has at least one fixed bed reactor (FBR, 17) connected upstream of it so that the at least one fixed bed reactor (FBR, 17) and the first fixed bed membrane reactor (PPBMR, 18) are connected in series.

5. Process according to Claim 4, **characterized in that** a gas stream (4) containing a mixture of at least one alkane and oxygen or an oxygen-containing gas is introduced into the at least one fixed bed reactor (FBR, 17).

6. Process according to Claim 4 or 5, **characterized in that** the gas stream (11) leaving the fixed bed reactor (FBR, 17) conversion containing at least one alkane, oxygen and at least one conversion product of the alkane is introduced into the interior of the tubular membrane into the fixed bed (18a) of the catalytic solid of the first fixed bed membrane reactor (PPBMR, 18).

7. Process according to any of the preceding claims, **characterized in that** at least one second fixed bed membrane reactor (CPBMR, 19) is connected in parallel with the serially connected fixed bed reactor (FBR, 17) and the first fixed bed membrane reactor (PPBMR, 18).

8. Process according to Claim 7, **characterized in that** at least one gas stream (3) containing at least one alkane is introduced into the fixed bed (19a) of catalytic solid of the second fixed bed membrane reactor (CPBMR, 19) and a gas stream (5) of oxygen or an oxygen-containing gas is introduced into the shell space (19b) of the second fixed bed membrane reactor (CPBMR, 19).

9. Process according to Claim 7 or 8, **characterized in that** the at least one gas stream (3) containing the at least one alkane which is introduced into the fixed bed (19a) of the second fixed bed membrane reactor (CPBMR, 19) is mixed with the gas mixture (strip phase, 7) discharged from the first fixed bed membrane reactor (PPBMR, 18) containing the at least one unconverted alkane, an unsaturated alkane product and oxygen before introduction into the fixed bed (19a) of the second fixed bed membrane reactor (CPBMR, 19).

10. Process according to any of Claims 7 to 9, **characterized in that** after flowing through and leaving the shell space (19b) of the second fixed bed membrane reactor (CPBMR, 19) as the shell-side gas stream (8) the gas stream (5) of oxygen or oxygen-containing gas introduced into the shell space (19b) of the second fixed bed membrane reactor (CPBMR, 19) is mixed with the gas stream (11) leaving the fixed bed reactor (FBR, 17) containing at least one alkane, oxygen and at least one conversion product of the alkane and this gas stream (12) is introduced into the interior of the tubular membrane into the fixed bed (18a) of the catalytic solid of the first fixed bed membrane reactor (PPBMR, 18) .

11. Process according to any of Claims 7 to 10, **characterized in that** after flowing through and leaving the shell space (19b) as the shell-side gas stream (8) of the second fixed bed membrane reactor (CPBMR, 19) the gas stream (5) of oxygen or oxygen-containing gas introduced into the shell space (19b) of the second fixed bed membrane reactor (CPBMR, 19) is introduced into the shell space (18b) of the first fixed bed membrane reactor (PPBMR, 18).

12. Reactor arrangement for performing a process according to any of the preceding claims **characterized by**
- at least one fixed bed reactor (FBR, 17),
- at least one first fixed bed membrane reactor (PPBMR, 18) comprising a tubular membrane having an interior as the reaction space, wherein the interior is filled with at least one catalytic solid (18a) as a fixed bed, an outer reactor shell surrounding the membrane and a shell space (18b) formed between the reactor shell and the membrane, wherein this first fixed bed membrane reactor (PPBMR, 18) is connected downstream of the fixed bed reactor (FBR, 17) so that the fixed bed reactor (FBR, 17) and the first fixed bed membrane reactor (PPBMR, 18) are connected in series and
- at least one second fixed bed membrane reactor (CPBMR, 19) connected in parallel with the serially connected fixed bed reactor (FBR, 17) and the first fixed bed membrane reactor (PPBMR, 18),
- wherein the at least one first fixed bed membrane reactor (PPBMR, 18) is coupled to the at least one second fixed bed membrane reactor (CPBMR, 19) via at least one first conduit, wherein via this first conduit the shell-side stream (7) from the first fixed bed membrane reactor (PPBMR, 18) is introduced into the conduit for the gas stream (3) containing at least one alkane for the second fixed bed membrane reactor (CPBMR, 19), and
- wherein a second conduit is provided between the two fixed bed membrane reactors (18,19), wherein via the second conduit the shell-side gas stream (8) from the second fixed bed membrane reactor (CPBMR, 19) is introduced into the shell side (18b) of the first fixed bed membrane reactor (PPBMR, 18) and/or into the conduit for the gas stream (11) leaving the fixed bed reactor (FBR, 17) into the catalytic fixed bed (18a) of the first fixed bed membrane reactor (PPBMR, 18).

13. Reactor arrangement according to Claim 12, **characterized in that** the first fixed bed membrane reactor (PPBMR, 18) and/or the second fixed bed membrane reactor (CPBMR, 19) comprise at least one tubular, gas-permeable membrane having a pore size of 2 nm to 500 nm, preferably 50 nm to 200 nm, for the first fixed bed membrane reactor (PPBMR, 18) or 2 nm to 20 nm for the second fixed bed membrane reactor (CPBMR, 19).

14. Reactor arrangement according to either of Claims 12 and 13, **characterized in that** the first fixed bed membrane reactor (PPBMR, 18) and/or the second fixed bed membrane reactor (CPBMR, 19) comprise at least one tubular, gas-permeable membrane coated with a silicon-containing compound.

15. Reactor arrangement according to Claim 14, **characterized in that** the material used to coat the membrane is a silicon-containing compound in particular based on colloidal silicon/silicon sols and/or a siliconoxycarbide compound.

## Revendications

1. Procédé pour la conversion par oxydation d'au moins un alcane, de préférence d'un C1-C4-alcane, en particulier de préférence du méthane, dans au moins un premier réacteur membranaire à lit fixe (PPBMR, 18) comprenant une membrane tubulaire pourvue d'un espace interne en tant qu'espace de réaction, l'espace interne étant rempli d'au moins un solide catalytique (18a) en tant que lit fixe, une enveloppe de réacteur externe entourant la membrane et un espace enveloppant (18b) formé entre l'enveloppe de réacteur et la membrane, **caractérisé en ce qu'**au moins un flux gazeux (2, 11, 12) contenant un mélange d'au moins un alcane et d'oxygène ou d'un gaz oxygéné est guidé dans l'espace interne de la membrane tubulaire dans le solide catalytique (18a) et en outre, au moins un flux gazeux (2a) contenant au moins un alcane est guidé dans l'espace enveloppant (18b).

2. Procédé selon la revendication 1, **caractérisé en ce que** ledit au moins un alcane est converti, en présence de l'oxygène pendant le passage à travers le solide catalytique (18a), au moins partiellement en un produit d'alcane insaturé et le mélange gazeux (7) ainsi formé, constitué d'alcane, d'oxygène et de produit d'alcane insaturé, étant évacué via la membrane hors du lit fixe catalytique (18a) et ainsi hors de l'espace de réaction dans l'espace enveloppant (18b).

3. Procédé selon la revendication 1 ou 2, **caractérisé en ce que** le rapport volumique d'alcane, en particulier de méthane, et d'oxygène dans le flux gazeux est de 1:10, de préférence de 1:5, en particulier de préférence 1:4.

4. Procédé selon l'une quelconque des revendications précédentes, **caractérisé en ce que** le premier réacteur membranaire à lit fixe (PPBMR, 18) est précédé d'au moins un réacteur à lit fixe (FBR, 17), de telle sorte que ledit au moins un réacteur à lit fixe (FBR, 17) et le premier réacteur membranaire à lit fixe (PPBMR, 18) sont commutés en série.

5. Procédé selon la revendication 4, **caractérisé en ce qu'**un flux gazeux (4) contenant un mélange d'au moins un alcane et d'oxygène ou d'un gaz oxygéné est guidé dans ledit au moins un réacteur à lit fixe (FBR, 17).

6. Procédé selon la revendication 4 ou 5, **caractérisé en ce que** le flux gazeux (11), contenant au moins un alcane, de l'oxygène et au moins un produit de conversion de l'alcane, quittant le réacteur à lit fixe (FBR, 17) est guidé dans l'espace interne de la membrane tubulaire dans le lit fixe (18a) constitué par le solide catalytique du premier réacteur membranaire à lit fixe (PPBMR, 18).

7. Procédé selon l'une quelconque des revendications précédentes, **caractérisé en ce qu'**au moins un deuxième réacteur membranaire à lit fixe (CPBMR, 19) est commuté en parallèle du réacteur à lit fixe (FBR, 17) et du premier réacteur membranaire à lit fixe (PPBMR, 18) commutés en série.

8. Procédé selon la revendication 7, **caractérisé en ce qu'**au moins un flux gazeux (3) contenant au moins un alcane est guidé dans le lit fixe (19a) constitué par le solide catalytique du deuxième réacteur membranaire à lit fixe (CPBMR, 19) et un flux gazeux (5) constitué par de l'oxygène ou un gaz oxygéné est guidé dans l'espace enveloppant (19b) du deuxième réacteur membranaire à lit fixe (CPBMR, 19).

9. Procédé selon la revendication 7 ou 8, **caractérisé en ce que** ledit au moins un flux gazeux (3) contenant ledit au moins un alcane, qui est guidé dans le lit fixe (19a) du deuxième réacteur membranaire à lit fixe (CPBMR, 19), est mélangé avec le mélange gazeux évacué du premier réacteur membranaire à lit fixe (PPBMR, 18) (phase d'extraction, 7) contenant ledit au moins un alcane non converti, un produit d'alcane insaturé et de l'oxygène avant l'introduction dans le lit fixe (19a) du deuxième réacteur membranaire à lit fixe (CPBMR, 19).

10. Procédé selon l'une quelconque des revendications 7 à 9, **caractérisé en ce que** le flux gazeux (5) introduit dans l'espace enveloppant (19b) du deuxième réacteur membranaire à lit fixe (CPBMR, 19), constitué par de l'oxygène ou un gaz oxygéné, après le passage dans et après avoir quitté l'espace enveloppant (19b) du deuxième réacteur membranaire à lit fixe (CPBMR, 19) est mélangé, en tant que flux gazeux côté enveloppe (8), avec le flux gazeux (11) quittant le réacteur à lit fixe (FBR, 17) contenant au moins un alcane, de l'oxygène et au moins un produit de conversion de l'alcane et ce flux gazeux (12) est guidé dans l'espace interne de la membrane tubulaire dans le lit fixe (18a) constitué par le solide catalytique du premier réacteur membranaire à lit fixe (PPBMR, 18).

11. Procédé selon l'une quelconque des revendications 7 à 10, **caractérisé en ce que** le flux gazeux (5) introduit dans l'espace enveloppant (19b) du deuxième réacteur membranaire à lit fixe (CPBMR, 19), constitué par de l'oxygène ou un gaz oxygéné, après le passage dans et après avoir quitté l'espace enveloppant (19b) est guidé, en tant que flux gazeux côté enveloppe (8) du deuxième réacteur membranaire à lit fixe (CPBMR, 19), dans l'espace enveloppant (18b) du premier réacteur membranaire à lit fixe (PPBMR, 18).

12. Disposition de réacteur pour la réalisation d'un procédé selon l'une quelconque des revendications précédentes, **caractérisée par**
- au moins un réacteur à lit fixe (FBR, 17),
- au moins un premier réacteur membranaire à lit fixe (PPBMR, 18) comprenant une membrane tubulaire pourvue d'un espace interne en tant qu'espace de réaction, l'espace interne étant rempli d'au moins un solide catalytique (18a) en tant que lit fixe, une enveloppe de réacteur externe entourant la membrane et un espace enveloppant (18b) formé entre l'enveloppe de réacteur et la membrane, ce premier réacteur membranaire à lit fixe (PPBMR, 18) étant disposé en aval du réacteur à lit fixe (FBR, 17) de telle sorte que le réacteur à lit fixe (FBR, 17) et le premier réacteur membranaire à lit fixe (PPBMR, 18) sont commutés en série et
- au moins un deuxième réacteur membranaire à lit fixe (CPBMR, 19) qui est commuté en parallèle du réacteur à lit fixe (FBR, 17) et du premier réacteur membranaire à lit fixe (PPBMR, 18) commutés en série,
- ledit au moins un premier réacteur membranaire à lit fixe (PPBMR, 18) étant relié audit au moins un deuxième réacteur membranaire à lit fixe (CPBMR, 19) via au moins une première conduite, le flux côté enveloppe (7) provenant du premier réacteur membranaire à lit fixe (PPBMR, 18) étant guidé à travers cette première conduite dans la conduite pour le flux gazeux (3) contenant au moins un alcane pour le deuxième réacteur membranaire à lit fixe (CPBMR, 19) et
- une deuxième conduite étant prévue entre les deux réacteurs membranaires à lit fixe (18, 19), le flux gazeux côté enveloppe (8) provenant du deuxième réacteur membranaire à lit fixe (CPBMR, 19) étant guidé via la deuxième conduite dans le côté enveloppe (18b) du premier réacteur membranaire à lit fixe (PPBMR, 18) et/ou dans la conduite pour le flux gazeux (11) quittant le réacteur à lit fixe (FBR, 17) dans le lit fixe catalytique (18a) dans le premier réacteur membranaire à lit fixe (PPBMR, 18) .

13. Dispositif de réacteur selon la revendication 12, **caractérisé en ce que** le premier réacteur membranaire à lit fixe (PPBMR, 18) et/ou le deuxième réacteur membranaire à lit fixe (CPBMR, 19) présente(nt) au moins une membrane tubulaire perméable aux gaz présentant une dimension de pore de 2 nm à 500 nm, de préférence de 50 nm à 200 nm pour le premier réacteur membranaire à lit fixe (PPBMR, 18) ou de 2 nm à 20 nm pour le deuxième réacteur membranaire à lit fixe (CPBMR, 19).

14. Dispositif de réacteur selon l'une quelconque des revendications 12 à 13, **caractérisé en ce que** le premier réacteur membranaire à lit fixe (PPBMR, 18) et/ou le deuxième réacteur membranaire à lit fixe (CPBMR, 19) présente(nt) au moins une membrane tubulaire perméable aux gaz, qui est revêtue par un composé contenant du silicium.

15. Dispositif de réacteur selon la revendication 14, **caractérisé en ce que** le matériau utilisé pour le revêtement de la membrane est un composé contenant du silicium, en particulier à base de silicium colloïdal ou d'un sol de silicium et/ou d'un composé d'oxycarbure de silicium.
